Europäisches Patentamt

European Patent Office

Office européen des brevets

(11) Publication number: **0 094 113**
**A2**

# EUROPEAN PATENT APPLICATION

(21) Application number: 83200598.7

(51) Int. Cl.³: **A 61 B 5/05**

(22) Date of filing: 26.04.83

(30) Priority: 30.04.82 GB 8212676

(43) Date of publication of application: 16.11.83
Bulletin 83/46

(84) Designated Contracting States: **AT BE CH DE FR GB IT LI LU NL SE**

(71) Applicant: **Brown, Brian Hilton, 9 Lorne Close, Dronfield Woodhouse Sheffield S18 5ZJ (GB)**
Applicant: **Barber, David Charles, 26 Westwood Road, Sheffield S11 7EY (GB)**
Applicant: **Freeston, Ian Leslie, 32 Rutland Park, Sheffield S10 2PB (GB)**
Applicant: **THE UNIVERSITY OF SHEFFIELD, Western Bank, Sheffield, S10 (GB)**

(72) Inventor: **Brown, Brian Hilton, 9 Lorne Close, Dronfield Woodhouse Sheffield S18 5ZJ (GB)**
Inventor: **Barber, David Charles, 26 Westwood Road, Sheffield S11 7EY (GB)**
Inventor: **Freeston, Ian Leslie, 32 Rutland Park, Sheffield S10 2PB (GB)**

(74) Representative: **Hulse, Thomas Arnold, Hulse & Co. Cavendish Buildings West Street, Sheffield S1 1ZZ (GB)**

(54) Tomography.

(57) Tomographic images ($I_1$ and $I_2$) of a body are constructed by placing a plurality of surface electrodes (1 to 16) at spaced intervals on the body (M), causing currents to flow in the body, and measuring the potential between pairs of electrodes, calculating the potential in each case on the assumption that the body consists of one uniform medium, plotting the isopotentials corresponding to the calculated results to create a uniform image of the body, obtaining the ratio between the measured potential and the calculated potential in each case, and modifying the image in accordance with the respective ratios by increasing the impedance along an isopotential in proportion to a ratio greater than unity or decreasing the impedance in proportion to a ratio less than unity. The calculations of potentials and the obtaining of ratios are carried out using a computer and the plotting of the isopotentials is carried out by a visual display unit (VDU) and/or a print-out unit run off the computer.

0094113

## TOMOGRAPHY

This invention relates to tomography and has for its object the provision of a method of construction of tomographic images of a body or mass (hereinafter referred to simply as a body), more particularly - but not exclusively - of any part of a live human body.

The success of X-ray computed tomography has encouraged the proposal of other medical imaging techniques not fraught with the dangers of X-rays. The use of low frequency electric currents has been suggested although no practical results have been published, but some literature exists on methods which involve measurements of resistance between electrodes on the surface of the body and propose methods for reconstruction of spatial resistivity variations, for example, "An Impedance Camera for Spatially Specific Measurements of the Thorax" by R. P. Henderson and J. B. Webster (I.E.E.E. Trans on Biomed. Eng. Vol.25, pp 250-254, 1978), "An Impedance Camera: A System for Determining the Spatial Variation of Electrical Conductivity" by R. J. Lytle and K. A. Dines (Lawrence Livermore Lab. Rep. U.C.R.L. 52413, 1978) and "Reconstruction of Spatial Resistivity Distribution of Conducting Objects from External Resistance Measurements by H. Schomberg (Philips GmbH, Hamburg, Mn MS-H, 1908V/78, 1978). However, the resolution problem is much greater than that involved in X-ray computed tomography because the current flow in tissue is not confined to the direct path between a pair of electrodes. L. R. Price has suggested in "Imaging of the Electrical Conductivity and Permittivity Inside a Patient: A New Computed Tomography (CT) Technique" (Proc. Soc. Photo-Opt. Instrum. Eng. (USA), Vol. 206, pp 115-119, 1979) and "Electrical Impedance Computed Tomography (ICT): A New CT Imaging Technique" (I.E.E.E. Trans. Nucl. Sci., Vol. NS-26, pp 2736-2739, 1979) a method which forces a sinusoidal spatial potential function on the conducting medium such that the current paths are parallel streamlines. The ratio of current to applied voltage is thus dependent on the line integral of conductivity and so standard tomographic reconstruction procedures can be used. However, R. H. T. Bates, G. C.

McKinnon and A. D. Seager have shown in "A Limitation on Systems for Imaging Electrical Conductivity Distributions" (I.E.E.E. Trans. on Biomed. Eng., Vol. 27, pp 418-420, 1980) that it is impossible to uniquely reconstruct images in this way unless ambiguities can be resolved by making extensive sets of measurements.

A major problem with any practical tissue resistance imaging method lies in the electrodes used to make contact. Electrode impedance is significant in comparison with tissue resistance at frequencies less than 100 kHz and operation at higher frequencies is extremely difficult because capacitive currents become significant. The impedance changes to be expected due to the spatial distribution of tissue resistivity are small and it is certainly necessary to be able to make measurements to an accuracy better than 1%. This is unlikely to be possible if electrode impedance is indistinguishable from tissue resistance.

According to the present invention, a method for the construction of tomographic images of a body comprises placing a plurality of surface electrodes at spaced intervals on the body, causing currents to flow in the body, and measuring the potential between pairs of electrodes, calculating the potential in each case on the assumption that the body consists of one uniform medium, plotting the isopotentials corresponding to the calculated results to create a uniform image of the body, obtaining the ratio between the measured potential and the calculated potential in each case, and modifying the image in accordance with the respective ratios by increasing the impedance along an isopotential in proportion to a ratio greater than unity or decreasing the impedance in proportion to a ratio less than unity.

The modifying of the impedance distribution in this manner is known as "back projection", and the execution of the back projection (or the superimposition of the modified impedance along isopotentials) results in a tomographic image of the distribution of impedance over the cross-sectional area of the body in the plane containing the electrodes. At low frequencies the impedances within the body may be purely resistive, because displacement currents are negligible in this situation, in which case the image is of resistivity rather than impedance, and - therefore - references in the foregoing and hereafter to "impedance" in relation to the body are to be regarded as embracing the alternative of "resistivity" at the appropriate frequences.

Currents may be caused to flow in the body either by applying an electrical potential between each pair of electrodes in turn or by electromagnetic induction.

The invention makes use of the fact that, whilst it is difficult to make an accurate direct measurement of resistance, it is possible to make precise measurements of potential via an electrode as long as the electrode impedance is very much smaller than the input impedance of the recorder.

The resolution of the tomographic image may be improved by iteration, by recalculating the potentials in each case using the modified impedance distribution as an approximate guide to the actual distribution of impedance, obtaining the ratio between the recalculated potential and the measured potential in each case, and modifying the modified impedance distribution accordingly. Alternatively, an image filter can be applied to correct for the point response function at all points within the back projected image.

The calculations of potentials and the obtaining of ratios may be carried out using a computer and the plotting of the isopotentials is carried out by a visual display unit (VDU) and/or a print-out unit run off the computer. Alternatively, the above calculations, iterations and filtering can be performed by electronic circuits specific for this purpose rather than by numerical calculations using an electronic computer.

One method of carrying out the invention and modifications thereof will now be described, by way of example only, with reference to the accompanying drawings, in which:-

Figure 1 is a diagram of an example using an array of sixteen surface electrodes equi-spaced round a body;

Figure 2 is a print-out tomographic image of one cross-section of a human arm ·

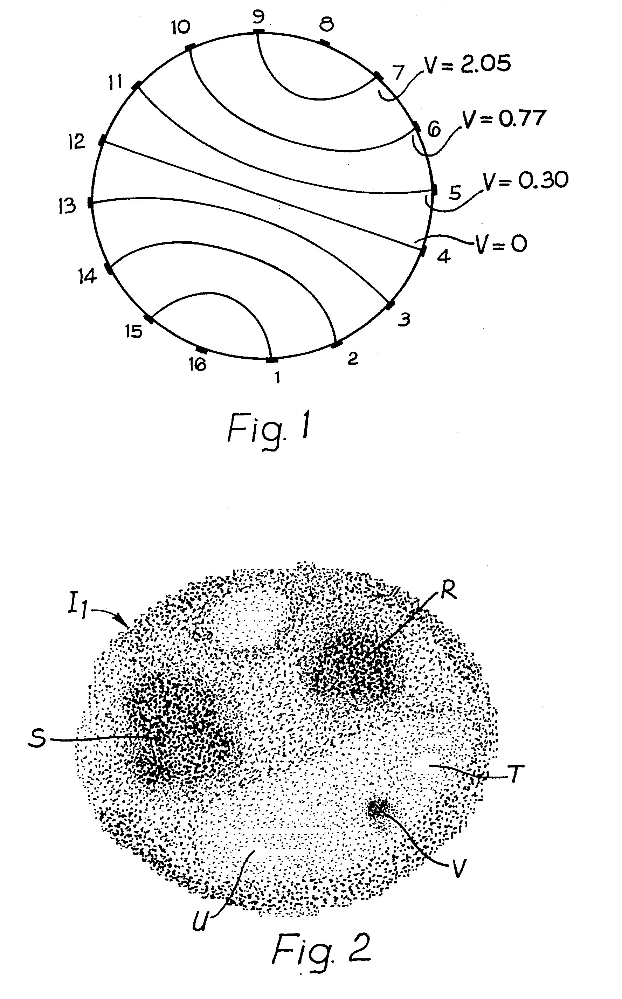

using the electrode array of Figure 1;

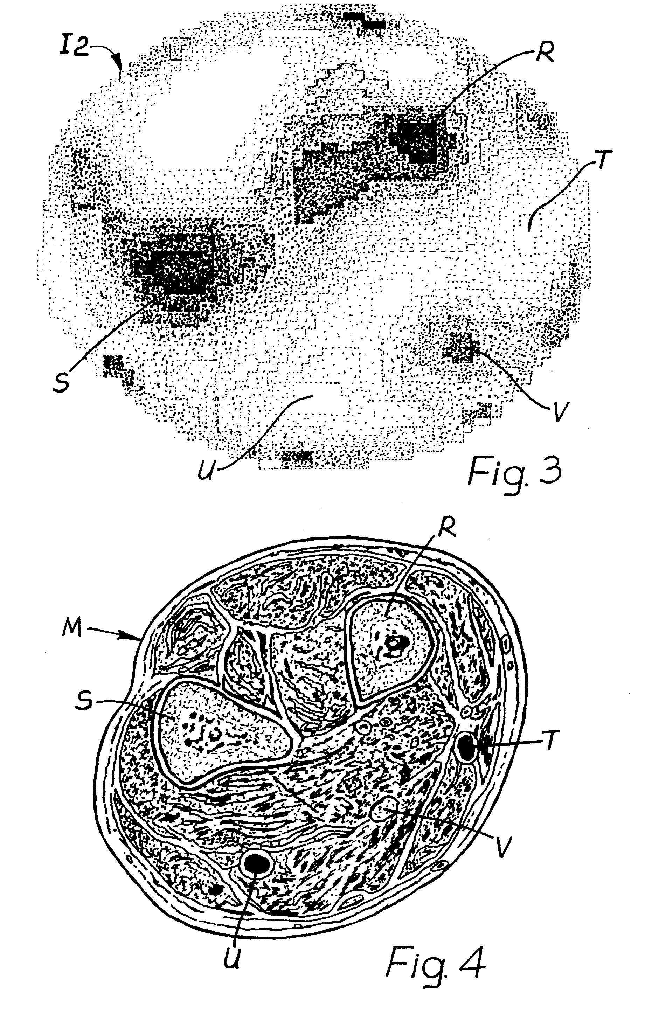

Figure 3 is the corresponding image on a visual display unit (VDU);

Figure 4 is the corresponding actual cross-section of the human arm;

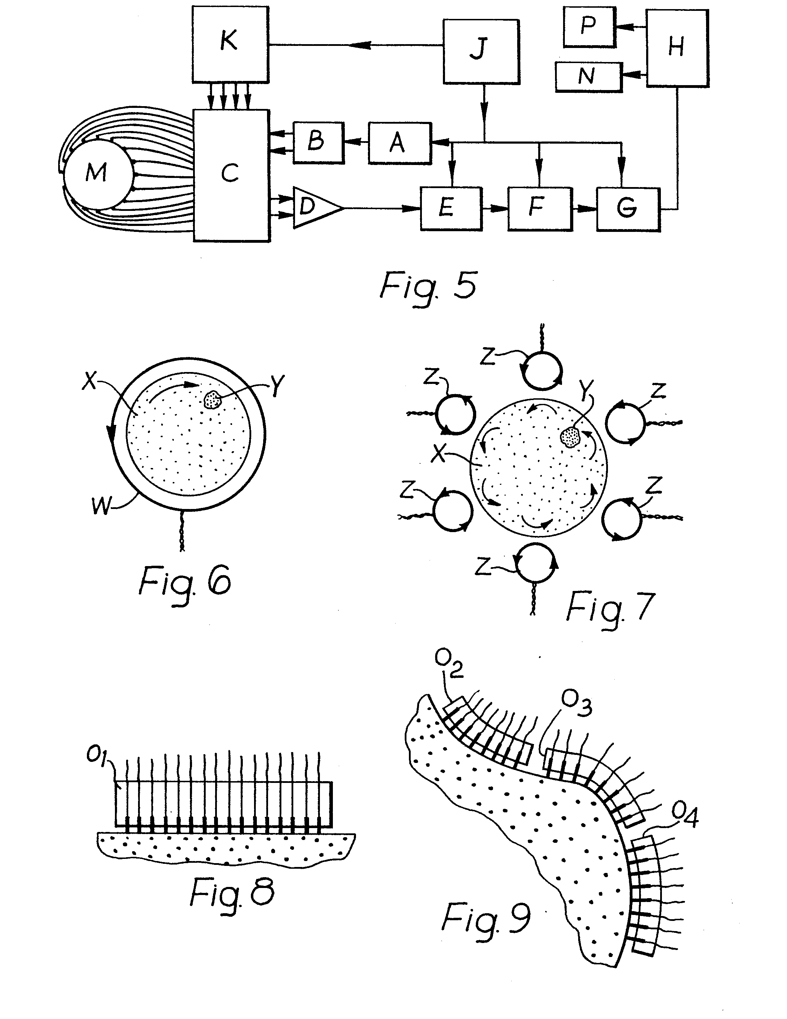

Figure 5 is a block circuit diagram of equipment used in conjunction with the electrode array of Figure 1 to produce the tomographic images of Figures 2 and 3;

Figures 6 and 7 are diagrams illustrating two ways of inducing currents in a body; and

Figures 8 and 9 are diagrams illustrating respectively a linear array of electrodes in a block, and the use of blocks of contoured arrays of electrodes.

Referring to Figures 1 and 5, an applied potential of approximately 3 volts at 4 milliamps is produced by a waveform generator A at 50kHz and applied through a voltage to current converter B and a multiplexer C in turn between every electrode combination and in each and every case the resultant potential between every adjacent pair of electrodes is fed through an amplifier D and a phase sensitive detector E and is recorded by a sample and hold unit F, from which the data is fed through a 12-Bit analogue to digital converter G to a computer H. The units A, E, F and G of the equipment are all controlled by a master clock J, which also controls the multiplexer through a unit K which stores the electrode combinations. The sixteen electrodes shown in Figure 1 give rise to 1456 potential measurements which can be recorded in 1.456 seconds or less.

Figure 1 also shows the isopotentials to be expected when current is applied between electrodes 8 and 16 on a body L assumed to consist of one uniform medium. In Figure 5 the sixteen electrodes are to be considered as being equi-spaced around a human arm M at the cross-section shown in Figure 4. The recorded potentials are compared by the computer H with the respective calculated potentials and the ratios are back projected along the appropriate isopotentials. Thus twelve or thirteen back projections can be made for every pair of current drive electrodes (a potential cannot be recorded from a current drive electrode) and the modified isopotentials plotted. The plots of the modified impedance along  isopotentials are superimposed on those obtained for each and every pair of drive electrodes, by means of the computer linked to a print-out N, to give a tomographic image $I_1$ as in Figure 2, and to a visual display unit (VDU) P, to give a visually displayed image $I_2$ as in Figure 3.

Comparing Figures 2, 3 and 4 it is possible to identify in the images $I_1$ and $I_2$ the radius and ulna bones R, S respectively, the radial and ulna arteries T, U respectively, and the median nerve V.  With greater resolution of the images more constituent parts of the arm M could be identified.

In addition to improving the resolution by iteration, the resolution can also be improved by  increasing the number of electrodes to say 32, but  this will call for more elaborate computing equipment to handle the increased number of recordings and calculations.

In  Figure  6  a  coil  W electromagnetically induces a current in a body X and an inhomogeneity Y causes surface potentials to be induced and which can be

0094113

picked up by electrodes disposed as in Figure 1 and processed by modified equipment as in Figure 5, while in Figure 7 currents are induced by a plurality of coils Z equi-spaced around the body X.

In Figure 8 a linear array of electrodes is mounted in a block $O_1$, while in Figure 9 blocks $O_2$, $O_3$, $O_4$ of contoured arrays of electrodes correspond to parts of the contour of a body.

The method of the invention can also be applied to tomographic image construction from three-dimensional data, but this involves taking into account the spread of current out of the plane of the electrodes and either back projection has to be made over isopotential surfaces, or the three-dimensional data reduced to two-dimensional format, which - again - calls for more elaborate computing equipment.

0094113

CLAIMS

1. A method for the construction of tomographic images of a body characterised by placing a plurality of surface electrodes at spaced intervals on the body, causing currents to flow in the body, and measuring the potential between pairs of electrodes, calculating the potential in each case on the assumption that the body consists of one uniform medium, plotting the isopotentials corresponding to the calculated results to create a uniform image of the body, obtaining the ratio between the measured potential and the calculated potential in each case, and modifying the image in accordance with the respective ratios by increasing the impedance along an isopotential in proportion to a ratio greater than unity or decreasing the impedance in proportion to a ratio less than unity.

2. A method as in Claim 1, characterised in that currents are caused to flow in the body by applying an electrical potential between each pair of electrodes in turn.

3. A method as in Claim 1, characterised in that currents are caused to flow in the body by electromagnetic induction.

4.  A method as in any one of Claims 1 to 3, characterised in that the resolution of the tomographic image is improved by iteration, by recalculating the potentials in each case using the modified impedance distribution as an approximate guide to the actual distribution of impedance, obtaining the ratio between the recalculated potential and the measured potential in each case, and modifying the modified impedance distribution accordingly.

5.  A method as in any one of Claims 1 to 3, characterised in that the resolution of the tomographic image is improved by applying an image filter to correct for the point response function at all points within the back projected image.

6.  A method as in any one of Claims 1 to 5, characterised in that the calculations of potentials and the obtaining of ratios is carried out using a computer and the plotting of the isopotentials is carried out by a visual display unit (VDU) and/or a print-out unit run off the computer.

7. A method as in Claim 5, characterised in that the calculations, iterations and filtering is performed by electronic circuits specific for this purpose.

8. A method as in any one of Claims 1 to 7, characterised in that a linear array of electrodes is mounted in a block.

9. A method as in any one of Claims 1 to 7, characterised in that blocks of contoured arrays of electrodes correspond to parts of the contour of a body.

10. A method as in any one of Claims 1 to 9, characterised in that the tomographic image is constructed from three-dimensional data, taking into account the spread of current out of the plate of the electrodes and either back projection being made over isopotential surfaces, or the three-dimensional data reduced to two-dimensional format.

1/3

Fig. 1

Fig. 2

*Fig. 3*

*Fig. 4*

Fig. 5

Fig. 6

Fig. 7

Fig. 8

Fig. 9